# EUROPEAN PATENT SPECIFICATION

(11) **EP 0 682 951 B1**
(45) Date of publication and mention of the grant of the patent: **17.12.1997**
(21) Application number: 95108885.5
(22) Date of filing: 31.05.1989
(51) Int. Cl.: A61K 39/21, G01N 33/569

(54) **Immunogenic compositions comprising a purified antigen constituted by the envelope glycoprotein of the HIV-2 EHO retrovirus**
Ein aus dem Hüll-Glycoprotein des HIV-2-EHO-Retrovirus bestehendes gereinigtes Antigen enthaltende immunologische Zusammensetzungen
Compositions immunogéniques comprenant un antigène constitué par la glycoprotéine d'enveloppe du rétrovirus HIV-2-EHO

(43) Date of publication of application: 22.11.1995
(62) Divisional of application: 89401498.4
(73) Proprietor: INSTITUT PASTEUR, 75724 Paris Cédex 15 (FR)
(72) Inventor: Hovanessian, Ara, F-93100 Montreuil (FR); Rey, Marie-Anne, F-75004 Paris (FR); Laurent, Anne, F-75007 Paris (FR); Krust, Bernard, F-75012 Paris (FR); Guetard, Denise, F-75015 Paris (FR); Montagnier, Luc, F-92350 Plessis-Robinson (FR)
(74) Representative: Desaix, Anne

(56) References cited:
- EP-A- 0 239 425
- WO-A-87/07906
- WO-A-88/05440
- WO-A-88/08449
- COMPTE RENDU DE L'ACADEMIE DES SCIENCES DE PARIS, vol. 302, no. 13, PARIS, pages 485-488, F CLAVEL ET AL 'LAV type II: un second rétrovirus associé au SIDA en Afrique de l'ouest'
- NATURE, vol. 326, 1987 LONDON GB, pages 662-669, GUYADER M. ET AL 'Genome organization and transactivation of the human immunodeficiency virus type 2'
- PROCEEDINGS OF THE NATIONAL ACADEMY OF SCIENCES OF USA, vol. 85, 1988 WASHINGTON US, pages 5941-5945, ZAGURY J.F. ET AL 'Genetic variability between isolates of human immunodeficiency virus (HIV) type 2 is comparable to the variability among HIV type 1'

## Description

Human immunodeficiency virus (HIV) is considered to be the etiologic agent of the acquired immunodeficiency syndrome (AIDS) (Montagnier et al. 1984). To date, two related but distinct types HIV-1 and HIV-2, have been identified (Barré Sinoussi et al 1983, Popovic et al, 1984, Ratner et al. 1985, Wain Hobson et al. 1985, Clavel et al 1986a, Brun-Vézinet et al, 1987).

HIV-2 has been described in european patent application n° 0239425 filed on January 22, 1987. At this time it was pointed out that HIV-2 is closely related to the simian immunodeficiency virus (SIV-mac), which causes an AIDS like disease in macaques. Alignments of the nucleotide sequences of HIV-1, HIV-2 and SIV reveal a considerable homology between HIV-2 and SIV-mac. These two viruses share about 75% overall nucleotide sequence homology, both of them are only distantly related to HIV-1 with about 40% overall homology see also (Guyader et al. 1987 ; Chakrabarti et al. 1977). In addition to the genes that encode structural proteins (the virion capsid and envelope proteins) and the enzymes required for proviral synthesis and integration common to all retroviruses, HIV-1, HIV-2 and SIV encode genes that regulate virus replication as well as genes that encode proteins to yet unknown function. The only notable difference in the genetic organizations of HIV-1, HIV-2 and SIV resides in the open reading frame referred to as vpx, which is absent in HIV-1 and vpu which is present in HIV-1 but not in HIV-2 and SIV (Cohen et al. 1988 ; Guyader et al. 1987). These viruses are both tropic and cytopathic for CD4 positive T lymphocytes (Klatzmann et al. 1984 ; Clavel et al. 1986a ; Dalgleish et al. 1984 ; Daniel et al. 1985). A great number of studies have indicated that CD4 functions as the cellular receptor of HIV (Weiss, 1988).

In other studies, it has been reported that HIV-2 and SIV mac can be differentiated from HIV-1 by the processing pathway of their envelope glycoprotein precursors. HIV-2 and SIV envelope precursors form an homologous dimer (which is a glycoprotein gp300 for HIV-2 ROD) during their processing into the mature products, the extracellular and the transmembrane glycoproteins gp125 and gp 36 respectively (Rey et al. J. Virol. 63/647.658 1989a). Furthermore, transmembrane glycoproteins of HIV-2 and SIV exist as homodimers having a molecular weight of about 80 kDa for HIV-2 ROD. Under similar experimental conditions, dimerization of the envelope precursor and dimeric forms of the transmembrane glycoprotein are not observed in the case of HIV-1. Therefore we can consider that dimerization of the envelope glycoproteins is a specific property of HIV-2 and SIV envelope gene expression. Accordingly, this property could be used as a convenient marker to differentiate between different isolates of HIV and SIV.

The inventors working further on the human HIV-2 retroviruses have now demonstrated that there could be some differences among the various isolates of HIV-2. In accordance with that they have defined subtypes of the retrovirus HIV-2.

A first subgroup can be related to the isolate HIV-2 ROD deposited with the CNCM under n° I 532 on February 21, 1986 and with the ATCC under n° 87011002 on January 9, 1987.

A second subgroup can be related to the isolate HIV-2 EHO, deposited with the CNCM under n° I 643 on December 19, 1986.

These two isolates have been described in the european patent application 0239425 and have been characterised by their common properties.

The inventors have been more particularly interested in studying the properties of a specific isolate of HIV-2 : HIV-2 EHO. According to the definition already given for the HIV-2 retrovirus type, HIV-2 EHO produces a 14kDa protein related to the product of the vpx gene of HIV-2 ROD.

The inventors have now pointed out a surprising property of HIV-2 EHO, related to the structure of its envelope precursor and to its mature products, the transmembrane and extracellular glycoproteins and corresponding proteins.

The invention is therefore directed to new antigens constituted by proteins and glycoproteins characteristic of the HIV-2 EHO subtype of the HIV-2 retrovirus.

The invention also concerns compositions comprising the above antigens and antibodies recognizing these antigens.

Moreover the invention provides new means for the in vitro diagnosis of an infection by a HIV-2 EHO retrovirus or a related retrovirus, involving these antigens.

The invention concerns also a process for the preparation of the antigens and antibodies cited above.

Surprisingly, the inventors have given evidence that the envelope precursor and the mature products, that is the extracellular and transmembrane glycoproteins of HIV-2 EHO which distinguish from the corresponding antigens of HIV-2 ROD by a difference in their molecular weights and the absence of cross reactivity between the HIV-2 EHO envelope glycoproteins or precursors or glycoproteins having the same immunological properties and, determined particular antibodies directed against HIV-2 ROD envelope glycoproteins or precursors of these glycoproteins.

The invention accordingly concerns a purified antigen of the human HIV-2 EHO retrovirus, characterised in that it is constituted by the envelope glycoprotein of HIV-2 EHO or a glycoprotein of a variant thereof or part of said antigen, or an antigen having the same immunological properties, which antigen is recognizable by human HiV-2 positive serum which is capable of recognizing the antigens of HiV-2 ROD, in an immunoprecipitation assay or in a western blot assay and it is not recognized by antibodies directed against the glycoprotein gp300 of HIV-2 ROD in the same conditions in an immunoprecipitation assay or in a western blot assay.

A typical human HIV-2 positive serum as mentioned hereabove, is a serum which is capable of recognizing the antigens of HIV-2 ROD.

The molecular weights of the various proteins and glycoproteins of the present invention could vary in a range of ±10% of the indicated molecular weight.

In a first aspect of the invention a purified antigen having the above characteristics is defined as a purified antigen of the HIV-2 EHO retrovirus or a glycoprotein having the same immunological properties and obtained from a variant of the HIV-2 EHO retrovirus , characterised in that it is encoded by the env gene and in that it has a molecular weight of the order of 120 kDa corresponding to the envelope precursor.

In a second aspect of the invention a purified antigen of the HIV-2 EHO retrovirus or a glycoprotein having the same immunological properties and obtained from a variant of the HIV-2 EHO retrovirus is characterised in that it is a glycoprotein gp270 corresponding to the dimeric form of the gp120 and in that it has a molecular weight of the order of 270 kDa.

This antigen represents the dimeric form of the precursor of the HIV-2 EHO glycoprotein gp120 which is firstly synthesized during the process of formation of the virus. This antigen seems to be necessary for the formation of the viral particles.

A third glycoprotein which is concerned by the invention is a purified antigen of the HIV-2 EHO retrovirus or a glycoprotein having the same immunological properties and obtained from a variant of the HIV-2 EHO retrovirus, characterised in that it corresponds to the mature product of the envelope precursor gp120 and in that it is the extracellular envelope glycoprotein gp100 having a molecular weight of the order of 100 kDa.

The characterisation by the inventors of the above glycoproteins related to the formation and maturation of the envelope glycoprotein gp100 of HIV-2 EHO, has enable them to conclude to a general difference of about 20 to 30kDalton (kDa) between the apparent molecular weights of the glycoproteins of HIV-2 EHO and the corresponding one of HIV-2 ROD.

They have further investigated to determine the origin of this difference. Since there are at least 30 potential N-linked glycosylation sites on the envelope precursor gp140 of HIV-2 ROD, it remained possible that the variation of the molecular weight between the glycoproteins related to the envelope glycoproteins of HIV-2 EHO on the one hand and of HIV-2 ROD on the other hand, be associated with a reduced number of oligosacharide chains in the above glycoproteins of HIV-2 EHO.

Surprisingly the inventors have demonstrated that the variations of the molecular weights of the various glycoproteins involved in the formation of the envelope of HIV-2 EHO is due to differences in the polypeptide patterns of the glycoproteins, which reflect differences in the amino-acid composition between both envelope precursors.

Therefore the invention also concerns a purified antigen of the HIV-2 EHO retrovirus or an unglycosylated product of the glycoprotein having the same immunological properties and obtained from a variant of the HIV-2 EHO retrovirus characterised in that it is a protein p60 and it has the same amino acids composition as the glycoprotein gp120 and in that it has no oligosaccharide chains.

In an other aspect of the invention any of the proteins derived from the above gp120, gp270, gp100 glycoproteins are concerned, providing that these proteins are free of the oligosaccharide chains present in the glycoproteins.

Such a protein could present particular epitopes which could usually not be accessible in the corresponding glycoprotein.

The above described proteins and glycoproteins can be included, alone or in any possible combination, in a composition for the in vitro diagnosis of an infection especially due to a human retrovirus of the HIV-2 EHO subtype.

In a first preferred embodiment, the composition defined hereabove is characterised in that it contains the precursor gp120 of the envelope glycoprotein, having a molecular weight of the order of 120 kDa.

In a second preferred embodiment the composition contains the extracellular envelope glycoprotein gp100, having a molecular weight of the order of 100 kDa, or it contains this gp100 in association with the above gp120.

In another embodiment according to the invention, the composition contains the dimeric glycoprotein gp270, having a molecular weight of the order of 270 kDa. This composition can further contains one of the above described glycoproteins or a combination thereof.

Other compositions which are in accordance with the definitions of the invention, are those containing proteins derived from the above glycoproteins and which are characterised by the absence of oligosaccharide chains.

One of the convenient proteins for the realisation of said compositions is the protein p60 above defined.

The invention also concerns a purified antigen of HIV-2 EHO in accordance with the above general definition, or a glycoprotein having the same immunological properties and obtained from a variant of HIV-2 EHO, characterised in that it is the transmembrane glycoprotein gp36, having a molecular weight, of the order of 36 kDa.

Another antigen according to the invention and which is related to the preceding one, or a glycoprotein having the same immunological properties, and obtained from a variant of HIV-2 EHO, is characterised in that it is a glycoprotein gp80 and it corresponds to the dimeric form of the gp36.

The invention also concerns an immunogenic composition comprising antigens of HIV-2 EHO as described in the preceding pages or antigens having the immunological properties of HIV-2 EHO antigens. Such composition could be used as vaccine. In this case they are prepared to allow the administration of a dose of 50 to 100µg per kilogram.

Compositions corresponding to the above definition comprise for example the glycoprotein gp100, which could be associated with others of the glycoproteins gp270, gp120, gp80 or gp36.

A preferred immunogenic composition according to the invention comprises further the corresponding antigens of HIV-2 ROD especially chosen among gp300, gp140, gp125, gp80 or gp36.

The invention also concerns polyclonal antibodies obtained from animals and monoclonal antibodies directed against the antigens defined hereabove.

The monoclonal antibodies according to the invention are, in a first embodiment, characterised in that they recognize a common epitope to the purified antigens gp120, gp270, gp100.

In a second embodiment these monoclonal antibodies are characterised in that they recognize specifically the glycoprotein gp100.

In a third preferred embodiment these monoclonal antibodies are characterised in that they recognize specifically the glycoprotein gp120.

In another preferred embodiment these monoclonal antibodies are characterised in that they recognize specifically the glycoprotein gp270.

More particularly the antibodies above described are such that they are directed against epitopes which are present on one or several antigens of the invention, these epitopes being not recognized by antibodies against the gp300 or one of the envelope related glycoproteins gp140 or gp125 of HIV-2 ROD.

Several processes for the production of antibodies can be employed according to the invention. For instance use can be made of ascitic cultures in animals, especially in rodents like mice or murine. Use can also be made of cellular cultures, either immobilysed or encapsulated or in suspension. The technique of the hybridomas obtained described by Kohler and Milstein (Nature 256 495-497) can be used. According to this, a process for preparing the hybridomas for the manufacture of the monoclonal antibodies of the invention comprises :
- starting from spleen cells of an animal, e.g. mouse or rat, previously immunized in vivo or from spleen cells of such animals previously immunized in vitro with an antigen of the invention.
- fusing said immunized cells with myeloma cells under hybridoma forming conditions and
- selecting those of the hybridomas which secrete the monoclonal antibodies which specifically recognize the above defined antigens.

The selection of the hybridomas can be realised by conventional techniques involving assays for the ability of hybridomas to produce antibodies against the determined antigens.

A process for producing the monoclonal antibodies directed against one or several antigens defined hereabove, comprises :
- culturing the selected hybridomas as indicated above in an appropriate culture medium and
- recovering the monoclonal antibodies excreted by said selected hybridomas, or alternatively
- implanting the selected hybridomas into the peritoneum of a mouse and, when ascites have been produced in the animal,
- recovering the monoclonal antibodies then formed from said ascites.

The antibodies can be recovered by various techniques such as radioimmuno-assay with the antigens to be recognized.

In another provision, the invention concerns a method for the preparation of a purified antigen having the above mentioned characteristics comprising the following steps :
- lysing the cells infected with a human retrovirus HIV-2 EHO or HIV-2 EHO viral pellets and separating the supernanant from the cellular extract,
- incubating the diluted extract with a serum of a patient infected with HIV-2 EHO or HIV-2 EHO viral pellets retrovirus or with polyclonal or monoclonal antibodies defined above, on an immunoaffinity column in conditions sufficient to allow the formation of a complex between said antibodies and antigens of the cellular extract,
- washing the immunoaffinity column to remove the molecules which are not retained on the support,
- resolving the eluted antigens.

The infected cells can be obtained in vitro after infection of cells for instance of CEM cells and after culture of these cells.

The resolution of the antigens can for instance by made by electrophoresis for example in polyacrylamide SDS gel.

The resolution of the proteins can for example be obtained by :
- elution of the proteins attached to the immunoaffinity column,
- purification of the products thus eluted on a chromatography column containing, bound to the support, monoclonal antibodies recognizing the antigen to be purified.

Preparation of these antigens can also be made with virus pellets.

In a preferred embodiment of the process for the preparation of the proteins, the lysis of the infected cells or virus pellets is realised in a detergent solution, the antibodies comprised in the immunoadsorbant are bound to agarose and a binding buffer is used.

The invention also concerns a process for the in vitro diagnosis of an infection specifically due to a HIV-2 EHO retrovirus in a biological sample detected for the possible presence of HIV-2 EHO, comprising the following steps:
- contacting the biological sample to be assayed, with one of several of the preceding antigen(s) or a composition containing the same, in conditions enabling the formation of a conjugate between said antigens and the antibodies possibly present in the biological sample,
- detecting the possibly formed immunological conjugate.

Another process according to the invention is a process for the in vitro diagnosis of an infection related to HIV-2 ROD or HIV-2 EHO subtype in a biological sample detected for the possible presence of antibodies against antigens of HIV-2 ROD or of HIV-2 EHO, comprising the following steps :
- contacting the biological sample to be assayed, with an antigen of HIV-2 EHO, or a composition of the antigens, in combination with the corresponding antigens of HIV-2 ROD which show some immunological differences with those of HIV-2 EHO as explained here above, in conditions enabling the formation of a conjugate between said antigens and the antibodies possibly present in the biological sample,
- detecting the possibly formed immunological conjugate.

A kit intended to be used according to the process for the in vitro diagnosis of a retrovirus of the HIV-2 EHO subtype in a biological sample detected for the presence of antibodies against HIV-2 EHO is characterised in that it comprises :
- an antigen having the properties defined above or a composition of these antigens possibly labeled,
- means enabling the reaction of formation of the immunological complex between the above antigens and the antibodies possibly present in the biological sample to be tested, when appropriate one or several incubation buffers,
- a negative control,
- means for the detection of the possibly formed immunological complex between said antigens and the antibodies possibly present in the sample.

Another kit for the diagnosis, is a kit for the in vitro diagnosis of an infection due to a retrovirus HIV-2 ROD or HIV-2 EHO in a biological sample detected for the presence of antibodies against HIV-2 ROD or HIV-2 EHO, characterised in that it comprises :
- antigens of HIV-2 EHO, as defined in the preceding pages in combination with the corresponding antigens of HIV-2 ROD, which present different immunological properties,
- means enabling the reaction for the formation of the immunological complex between the above antigens and the antibodies possibly present in the biological sample to be tested, when appropriate one or several incubation buffers,
- a negative control,
- means for the detection of the possibly formed immunological complex between said antigens and the antibodies possibly present in the sample.

The step of detection of the possibly formed immunological complex can be made by conventional methods such as Western blot or ELISA.

Preferred compositions of antigens which enable the diagnosic of HIV-2 according to the invention are for example those comprising a mixture of one or several antigens chosen among gp300, gp140, gp125, gp80 or gp36 of HIV-2 ROD with one or several antigens chosen among gp270, gp100, gp80 or gp36 of HIV-2 EHO.

The present divisional application relates specifically to immunogenic compositions as defined above.

Further advantages of the invention will appear in the examples and figures which follow.

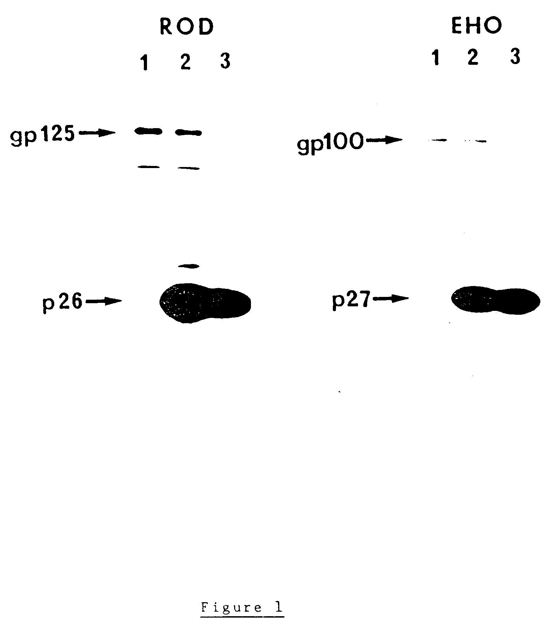

### FIGURE 1 : Identification of HIV-2 EHO proteins by immunoprecipitation.

HIV-2 ROD or EHO infected CEM cells were labeled with [³⁵S] methionine and the culture supernatant was used in an immunoprecipitation assay using serum from the patient EHO (lanes 1), a typical HIV-2 positive serum (lanes 2) and a typical HIV-1 positive serum (lanes 3). Samples were analyzed by polyacrylamide gel (12,5%) electrophoresis. A fluorograph is shown. gp125 and p26 refer to the extracellular envelope glycoprotein and the major core protein of HIV-2 ROD, respectively. gp100 and p27 refer to the extracellular envelope glycoprotein and the major core protein of HIV-2 EHO, respectively.

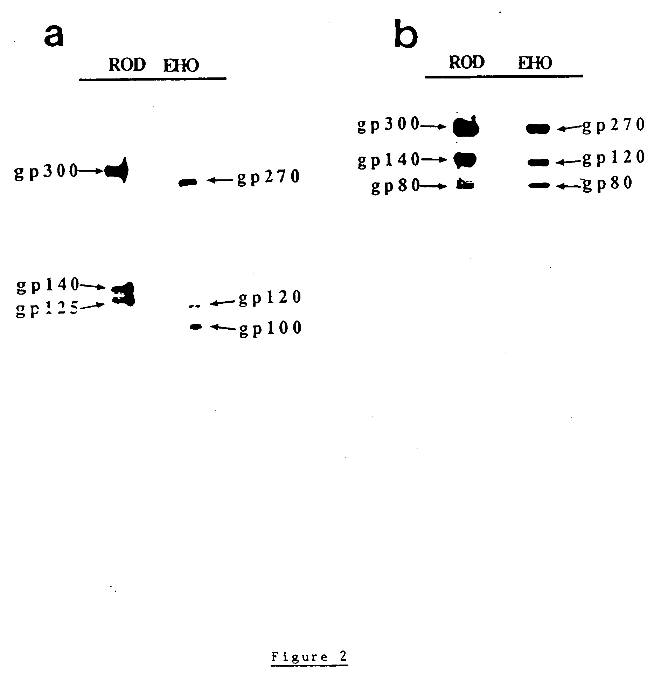

### FIGURE 2 : Characterization of HIV-2 EHO envelope glycoproteins.

a/ HIV-2 ROD and EHO infected cells were labeled with [³⁵S] methionine and extracts were then used in an immunoprecipitation assay using a typical HIV-2 positive serum. The samples were analyzed by 5% polyacrylamide gel containing 0,1% bis-acrylamide.
b/ Extracts from HIV-2 infected cells labeled with [³H] glucosamine were assayed by immunoprecipitation using a monoclonal antibody specific for the transmembrane glycoprotein of HIV-2 ROD. The samples were analyzed by polyacrylamide gel (10%) electrophoresis.

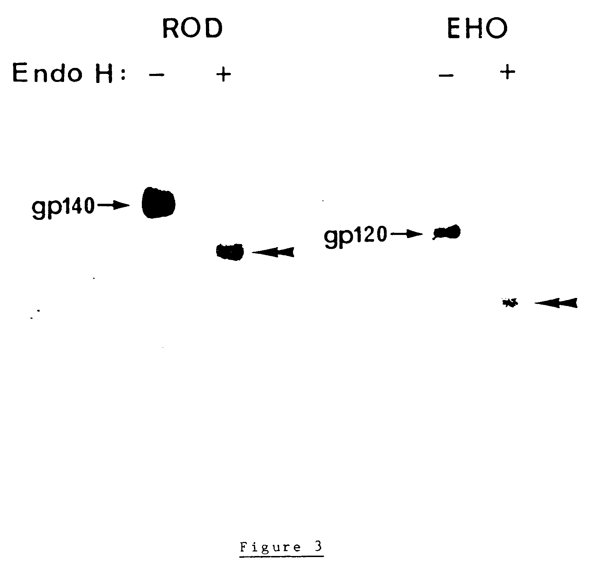

### FIGURE 3 : Electrophoretic mobilities of deglycosylated envelope precursors of HIV-2 ROD and EHO.

[³⁵S] methionine labeled gp300 (ROD) and gp270 (EHO) were purified by immunoprecipitation followed by preparative gel electrophoresis ("Materials and Methods"). The lyophilized samples were suspended in the endo H buffer (which results in the dissociation of dimers) containing 150mM sodium citrate pH 5.5, 0.1% SDS (w/v) and 0.5mM PMSF. These samples were incubated (30°C 2h) in the absence (lanes-) or presence (lanes +) of 10 units of endo H. Reactions were stopped by the addition of 2 fold concentrated electrophoresis sample buffer. Samples were analyzed by polyacrylamide gel (7.5%) electrophoresis. Fluorographs are presented. On the right the arrows indicate the position of the digested products, 80 and 60 kDa for ROD and EHO, respectively.

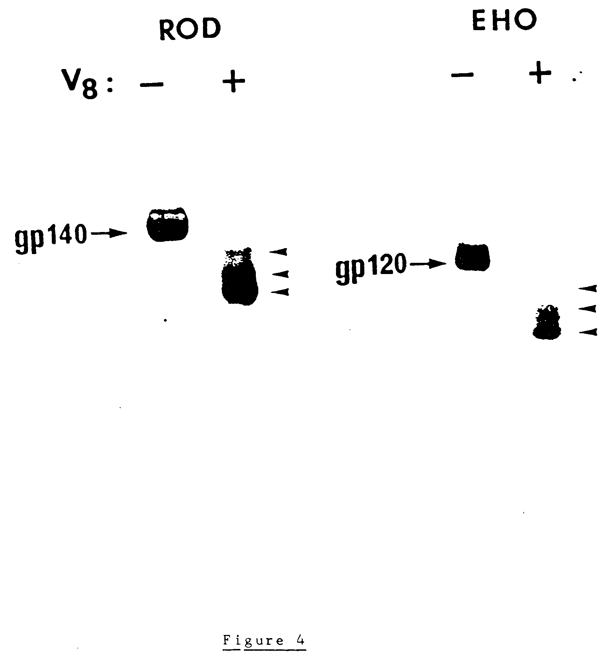

### FIGURE 4 : Partial digestion of HIV-2 ROD and EHO envelope precursors with the V8 protease.

[³⁵S] methionine labeled envelope precursors from HIV-2 ROD and EHO were purified as described in Figure 3. The lyophilized samples were suspended in 100 µl of buffer containing 62.5mM Tris-HCl pH 6.8 and O.1% SDS (w/v). The samples were then incubated (37°C, 30 min) without (lanes -) or with (lanes +) 1µg of staphyloccocus aureus V8 protease. Reactions were stopped by the addition of two fold concentrated electrophoresis sample buffer. The different samples were analyzed by electrophoresis in a 7.5% polyacrylamide gel. A fluorograph is shown. On the right the arrows indicate the position of the digestion products : 130, 110 and 80 kDa for gp140 ROD whereas 90, 75 AND 60 kDa for gp120 EHO.

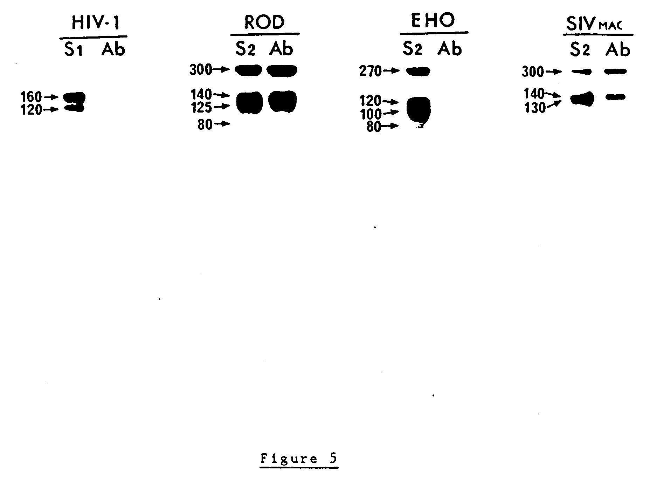

### FIGURE 5 : Immunoprecipitation assay using polyclonal antibodies raised against gp300 of HIV-2 ROD.

[³H] glucosamine labeled extracts from HIV-1 BRU, HIV-2 ROD, HIV-2 EHO and SIV mac infected cells were assayed by immunoprecipitation using a typical human HIV-1 positive serum (lane S1), a typical human HIV-2 positive serum (lanes S2) and murine polyclonal antibodies raised against gp300 of HIV-2 ROD (lanes Ab). Samples were analyzed by polyacrylamide gel (12.5%) electrophoresis. Fluorographs are shown.

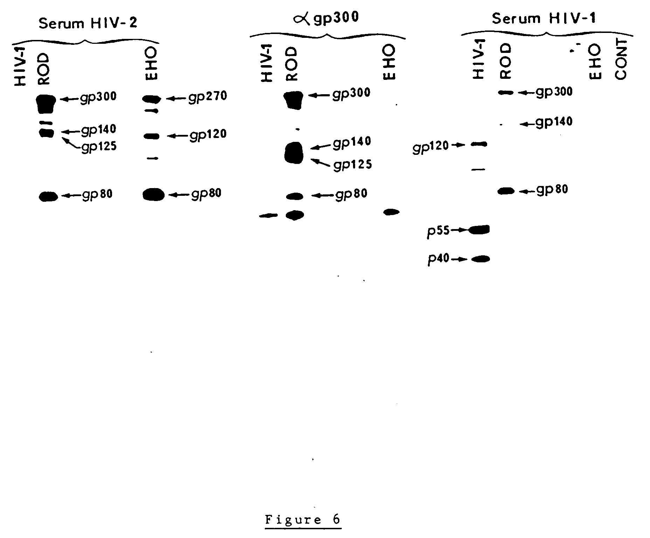

### FIGURE 6 : Envelope glycoproteins of HIV-2 EHO are not recognized by anti-gp300 antibodies.

Extracts from CEM cells infected with HIV-1 or HIV-2 ROD or HIV-2 EHO were analyzed by immunoblotting using the serum of the patient EHO (section Serum HIV-2), murine anti-gp300 antibodies (section anti-gp300) and a typical HIV-1 positive serum (section Serum HIV-1). CONT refers to extracts from uninfected cells ; Polyacrylamide gel electrophoresis was carried out in 10% acrylamide (in sections Serum HIV-2 and Serum HIV-1) or 7.5% polyacrylamide (sections anti-gp300) gels. Autoradiographs are presented.

### MATERIALS AND METHODS

### MATERIALS

L-[³⁵S] Methionine (specific activity > 1000 µCi/mmol), D-[6-³H] Glucosamine (specific activity : 20-40 µCi/mmol) were purchased from Amersham (Amersham, UK). Endo-β-N-acetylglucosaminidase H and Staphylococcus V8 protease were from Calbiochem, San Diego, U.S.A. Poly(A).poly(U) was obtained at Institut Curie, Paris, and prepared according to the method described by Hovanessian et al. 1982 (Journal Interferon Research vol. 2 p. 209 à 216).

The monoclonal antibody specific for the transmembrane glycoprotein was obtained from Oncogen Seattle, U.S.A. The preparation of this monoclonal antibody (referred to as mAb1H8) is described.

### VIRUS AND CELLS

HIV-1_{BRU} isolate of the human immunodeficiency virus type 1 (Montagnier et al. 1984), HIV-2ROD isolate of the human immunodeficiency virus type 2 (Clavel et al., 1986) and Simian immunodeficiency virus, SIVmac₁₄₂ (Daniel et al., 1985) were used in this study.

The different cell lines and human lymphocytes were cultured in suspension medium RPMI-1640 (GIBCO-BRL, Cergy-Pontoise France) containing 10% (v/v) fetal calf serum ; 2µg/ml polybrene (Sigma) was added for HIV infected cell cultures. CEM clone 13 cells are derived from the human lymphoid cell line CEM (ATCC-CCL 119) and express the T4 antigen to a high level. Five days after infection with HIV-1_{BRU} or HIV-2_{ROD} isolates, about 80-90% of the cells produce viral particles and can be identified by a cytopathic effect corresponding to vacuolisation of cells and appearance of small syncitia.

The HUT-78 cell line is another human T4 positive lymphoid cell line that is highly permissive for the replication of SIVmac₁₄₂ (Daniel et al., 1985). Peripheral blood lymphocytes from healthy blood donors were stimulated for three days with 0,2% (w/v) phytohemagglutinin (PHA) fraction P (Difco, Detroit, USA) in RPMI-1640 medium supplemented with 10% fetal calf serum. Cells were then cultured in RPMI-1640 medium containing 10% (v/v) T cell growth factor (TCGF, Biotest). After infection with HIV-2, lymphocytes were cultured in presence of 10% (v/v) TCGF and 2µg/ml Polybrene.

The HIV-2 EHO isolate was obtained from blood lymphocytes of Ivory Coast patient (EHO) with AIDS. Blood lymphocytes were stimulated with PHA and cocultured with PHA-stimulated, normal human lymphocytes. These cultures were maintained in the presence of TCGF. The production of virus was monitored by cytopathic effects and by the reverse transcriptase activity in the supernatant (Rey et al. 1987). The virus (HIV-2 EHO) was then cultured on CEM clone 13 cells. Four to five days after infection with HIV-2 EHO approximately more than 80% of cells produced viral particles.

### METABOLIC LABELING OF CELLS

For metabolic labeling of proteins, infected cells were incubated for 16 hours at 37°C in MEM culture (Milieu Minimum Essentiel, abréviation de Minimum Essential Medium) without L-methionine and serum but supplemented with 200 µCi/ml[³⁵] methionine. For metabolic labeling of glycoproteins, infected cells were incubated for 16 hours at 37°C in MEM culture medium lacking serum and glucose but supplemented with 200 µCi/ml [³H]glucosamine.

### CELLS AND VIRAL EXTRACTS

Cell pellets corrresponding to 10⁷ cells were resuspended in 100 µl of buffer : 10mM Tris-HCl pH 7.6, 150mM NaCl, 1mM EDTA, 0.2mM PMSF, 100 units/ml aprotinin (Iniprol®, Choay) before addition of 100 µl of the same buffer containing 2% (v/v) Triton X-100®. Cells extracts were centrifuged at 12,000g for 10 minutes, and the supernatant was stored at -80°C until used. For viral extract preparations, 100µl of 10X lysis buffer (100mM Tris-HCl pH 7.6, 1.5M NaCl, 10mM EDTA, 10% 5v/v) Triton X-100®, 100 units/ml aprotinin) was added per ml of clarified supernatant from infected CEM cells and processed as above. For the preparation of extracts from virus pellets, culture medium from infected cells was first centrifuged at 12,000g for 10 minutes before high speed centrifugation at 100,000g for 15 min in a Beckman TL100 centrifuge. Virus pellets (material from 10⁷ cells) were then solubilized in 200µl of lysis buffer.

### PREPARATIVE ELECTROPHORESIS

HIV-2 glycoproteins purified by immunoprecipitation were resolved by polyacrylamide gel electrophoresis as previously described (Rey et al. 1989 J.Virol 63, 647-658) and the regions of the gel containing the viral glycoproteins were cut out by reference to the position of prestained molecular weight protein markers (BRL).

Glycoproteins were eluted by incubation for 16 hours at 4°C in elution buffer (0.1M NaHCO₃, 0.5mM EDTA, 0.05% (w/v) SDS, 0.2mM PMSF). The glycoprotein fractions thus obtained were lyophilized and kept refrigerated until used.

### PREPARATION OF MURINE POLYCLONAL ANTIBODIES, ANTI-gp300

HIV-2 ROD envelope glycoprotein gp300 was purified from extracts of infected CEM cells (3 x 10⁸ cells) by immunoaffinity chromatography on the HIV-2 serum-Sepharose® and followed by preparative gel electrophoresis (Rey et al. 1989a précité). The purified preparation of gp300 was dissolved in 10 ml of 150mM NaCl containing 0.5M urea and 1 mg/ml of mouse serum proteins and dialyzed for 24 hours against the solution containing 150 mM NaCl and 0.5M urea. The dialized material was then centrifuged and 2ml aliquots were stored at -80°C. Five mice (6 weeks old) were injected intraperitoneally, five times at 12 days interval with 350 µl of the gp300 preparation (about 0.1 µg of gp300). Poly(A) Poly (U) (200 µg ; 1 mg/ml in 150 mM NaCl) was used as an adjuvant which was administered intravenously during each immunization. Two days after the last injection mice were injected intraperitoneally with a suspension of 10⁶ sarcoma 180/TG cells to prepare hyperimmune ascitic fluid (Hovanessian et al. 1988 Immunology Today 9, 161-162). Eight days later mice were sacrificed and the ascitic fluids were collected. Ascitic cells were removed by centrifugation (200g, 5 min) and the peritoneal fluid was collected.

### PRODUCTION AND CHARACTERIZATION OF MONOCLONAL, mAb 1H8

HIV-2 ROD virions were cultivated in CEM cells and purified from concentrated culture supernatants by banding in sucrose gradients. Purified virus was disrupted in 0.5% Triton X-100®, 150 mM NaCl, 50mM Tris, pH 8.0, 1% aprotinin (Sigma) and clarified by ultra-centrifugation. The viral extract was then passed over a Lentil-Lectin Sepharose® 4B affinity column (Pharmacia), the column washed, and the bound glycoproteins eluted with 0.5 M methyl α-D-mannopyranoside (Sigma), and dialyzed overnight against phosphate-buffered saline. BALB-C mice were immunized intraperitoneally with 0.3 ml of purified glycoproteins (2-5µg) reattached to Lentil-Lectin Sepharose® 4B (50-100 µl). The mice were boosted every 4-6 weeks for 24 weeks with the same immunogen and monitored for HIV-2 virion extracts and quantitatively on the Genetic Systems HIV-2 disrupted virion EIA. Three days after the last injection, spleen cells were fused with NSl myeloma cells according to the method of Kohler and Milstein. 96-well fusion plates were screened for hybridomas secreting anti-HIV-2 antibodies using the Genetic Systems HIV-2 disrupted virion EIA. Methods for the propagation and stabilization of clones hybridomas and for ascites production have been previously described Gosting et al (J. of Clinical Microbiology 25, 845-848). Hybridoma culture supernatants were screened by RIPA and Western blot analysis. Monoclonal antibody (mAb) lH8, which reacted with the transmembrane glycoprotein, was further mapped to amino-acid sequence 579-604 within the HIV-2 transmembrane glycoprotein using a synthetic peptide based EIA. The HIV-2 amino-acid sequence 579-604 is highly conserved among all HIV-2 ans SIV isolates thus far sequenced accordingly, monoclonal antibody lH8 cross-reacts with all HIV-2 and SIV isolates thus far tested. The synthetic peptide p39' was synthesized according to the amino-acid sequence 579-604 deduced from the nucleotide sequence of the HIV-2 ROD envelope. The amino-acid sequence of peptide p39' is the following VTAIEKYLQDQARLNSWGCAFRQVCH.

### RADIO-IMMUNOPRECIPITATION ASSAY (RIPA)

Cell or viral extracts (20µl) (material corresponding to 1 x 10⁶ infected cells) were first diluted in two volumes of RIPA buffer [(10mM Tris-HCl pH 7.6, 150mM NaCl, 1mM EDTA, 1% Triton X-100® (v/v), 0,2% sodium deoxycholate (wt/v), 0.1% SDS (wt/v) 7 mM 2 β-mercaptoethanol, 0.2 mM PMSF, 100 units/ml of aprotinin (Iniprol®, Choay)]. Diluted extracts were incubated (45 min, 4°C) with sera or with murine polyclonal or monoclonal antibodies (2-5µl). Protein A-Sepharose® was then added and the samples were further incubated for 3 h at 4°C. These samples were washed in the RIPA buffer. Proteins recovered by immunoprecipitation were eluted by heating (95°C, 5min) in the electrophoresis sample buffer [125 mM Tris-HCl, pH 6.8, 1% SDS (wt/v), 20% glycerol (v/v), 1% 2 β-mercaptoethanol]. Eluted proteins were resolved by electrophoresis in polyacrylamide SDS gels containing O.1% bis-acrylamide instead of 0.2% (wt/v) and 2M urea.

### ELECTROPHORETIC TRANSFER IMMUNOBLOT ANALYSIS:WESTERN BLOT

Proteins were subjected to analysis by polyacrylamide gel electrophoresis before being electrophoretically transferred to 0.45 µm nitrocellulose sheets (Schleicher and Schüll, Dassel, FRG) in electrode buffer (20mM Tris base, 150mM glycine, 20% methanol, v/v) as described (Burnette 1981, Anal. Biochem. 112, 195-230). The electrophoretic blots were saturated with 5% (w/v) non-fat dry milk in PBS (Johnson et al. 1984 Gene Anal. Techn. l, 3-8). They were then incubated in a sealed bag (overnight 4°C) either with HIV-1 or HIV-2 positive sera (at 1 : 100 dilution) or with mouse polyclonal or monoclonal antibodies (at 1: 200 dilution) in PBS containing 10% FCS. The sheets were subsequently washed in PBS, PBS containing 5% Nonidet P-40 and then resaturated in PBS containing nonfat milk (5%). The washed sheets were then incubated (2 hr, room temperature) in a sealed bag either with a preparation of ¹²⁵I-labeled protein A (Amersham, >30mCi/mg) to reveal the human polyclonal antibodies in the HIV-1 or HIV-2 sera or with a preparation of ¹²⁵I-labeled goat anti-mouse immunoglobulins (Amersham ; 2-10 µCi/ µg). The sheets were removed from the bags and washed again, dried and autoradiographed (Kodak RP Royal, X-Ray films) to 24-48 hr.

### RESULTS

### ISOLATIONS OF HIV-2 EHO :

HIV-2 EHO was isolated from the peripheral blood lymphocytes of an AIDS patient (EHO) from Ivory Coast. Virus was isolated by coculturing PHA-stimulated lymphocytes from the patient EHO and PHA stimulated normal human lymphocytes in the presence of TCGF. Reverse transcriptase activity in the culture supernatant was detectable at 15 days associated with a typical cytopathic effect. The virus was then propagated on CEM cells by coculturing with infected lymphocytes. Supernatants of such cultures were then used to infect CEM cells and prepare the stock of virus used in the experiments discussed here. This virus belongs to the HIV-2 retrovirus type since in a dot-blot assay its genomic RNA hybridized with a probe from HIV-2 ROD but not from HIV-1 BRU.

The stock preparation of HIV-2 EHO infects CEM cells and causes a cytopathic effect. Three to four days after infection of CEM cells with HIV-2 EHO, about 80 to 90% of cells produced viral proteins detectable by immunofluorescence studies using an HIV-2 positive serum. Four days after infection most of the cells showed a clear cytopathic effect corresponding to vacuolization of cells and appearance of small syncitia.

In order to characterize the proteins of HIV-2 EHO virus, infected cells were metabolically labeled with [³⁵S]methionine and the viral pellet in the culture supernatant was assayed by immunoprecipitation using different sera :
1/ the serum of the patient infected by HIV-2 EHO;
2/ a typical HIV-2 positive serum;
3/ a typical HIV-1 positive serum.

These assays were carried out in parallel with extracts of [³⁵S]methionine labeled HIV-2 ROD virus. The HIV-2 positive serum and the serum of the patient EHO both recognized the extracellular glycoprotein gp125 of HIV-2 ROD. However, only the HIV-2 positive serum recognized the major core protein p26 of HIV-2 ROD (Figure 1, lanes 1 and 2, ROD). The HIV-1 positive serum did not recognize gp125 but it could immunoprecipitate p26 (Figure 1, lane 3, ROD). This is in accord with previous results indicating that the gag proteins of HIV-1 and HIV-2 are antigenically cross-reactive whereas the env proteins are not (Clavel et al. 1986a). The reactivity of these three sera with the viral proteins of HIV-2 EHO was similar to that of HIV-2 ROD. The serum of the patient-EHO and the HIV-2 positive serum immunoprecipitated a 100 kDa protein which was presumed to be the extracellular envelope glycoprotein of HIV-2 EHO (Figure 1, lanes 1 and 2 EHO). This 100 kDa protein was specific to HIV-2 since it was not recognized by the HIV-1 positive serum. Both HIV-1 and HIV-2 positive sera precipitated a 27 kDa protein which was probably the major core protein of HIV-2 EHO. However, this latter protein was not recognized by the serum from patient EHO (Figure 1, EHO). These observations indicate the absence of anti-gag antibodies in the serum of patient EHO. Such a phenomenon has been reported in HIV-1 infected individuals and it is associated with the developement of AIDS. The late stages of HIV-1 infection are characterized by an increased production of antigens and a decreased level of antibodies directed against the major core protein. In fact this observation is routinely used as a marker to monitor the switch from latent to active HIV infection (Lange et al., 1986 Br. Med. J.293, 1459-1462; Pedersen et al., 1987 Br. Med. J. 295, 567-572).

### IDENTIFICATION OF EXTERNAL AND TRANSMEMBRANE ENVELOPE PROTEINS OF HIV-2 EHO

In these experiments, HIV-2 ROD and EHO infected CEM cells were metabolically labeled with [³⁵S]methionine and the labeled extracts were assayed by immunoprecipitation using different types of antibodies : a typical HIV-2 positive serum which identifies envelope proteins of HIV-2 ROD (Rey et al. 1989a), a monoclonal antibody specific for the transmembrane glycoprotein of HIV-2 ROD, a monoclonal antibody specific for the major core protein p26 of HIV-2 ROD and rabbit polyclonal antibodies specific for the vpx protein.

In the case of HIV-2 ROD envelope glycoproteins, it has been previously shown that the envelope precursor (gp140) forms a dimer (gp300) during its processing, required for the production of mature envelope proteins : the extracellular glycoprotein gp125 and the dimeric form of the transmembrane glycoprotein gp80. The monomer of the transmembrane glycoprotein is rarely detectable under the experimental conditions used in our experiments. In order to compare the molecular weight of the envelope glycoproteins an electrophoresis was performed in a 5% polyacrylamide gel containing 0.1% bis-acrylamide instead of 0.2%. Figure 2a shows the results of this experiment indicating that the envelope precursor, the dimeric form of the envelope precursor and the extracellular envelope glycoprotein are 20-30kDa smaller in HIV-2 EHO compared to HIV-2 ROD. In order to identify the transmembrane glycoprotein of HIV-2 EHO we used a monoclonal antibody mab1H8 against the transmembrane glycoprotein of HIV-2 ROD. As it is expected, this monoclonal antibody identifies also the envelope precursor and its dimeric form. Accordingly, the monoclonal antibody immunoprecipitated gp120, gp270 and gp80 from HIV-2 EHO infected cells compared to gp140, gp300 and gp80 from HIV-2 ROD infected cells (Figure 2b). The electrophoretic mobility of the transmembrane glycoprotein dimer of HIV-2 EHO was slightly slower than that of HIV-2 ROD. Since this difference is not significant, for convenience we will refer to it as gp80. Pulse-chase experiments and studies on the kinetics of accumulation of envelope proteins of HIV-2 EHO, indicated that gp120 was the first glycoprotein synthetized before the formation of the precursor dimer gp270 and the production of mature envelope proteins : extracellular glycoprotein gp100 and the transmembrane glycoprotein dimer gp80. Glycosylation of all envelope proteins was blocked by tunicamycin, an antibiotic which inhibits N-linked glycosylation of proteins (Schwartz et al. 1976 J.Virol. 19, 782-791 ; Kornfeld and Kornfeld 1985, Ann.Rev.Biochem. 54, 631-664). Among these glycoproteins of the envelope only gp100 and gp80 were found to be associated with virus particles according to previously reported results on HIV-2 ROD (Rey et al., 1989a).

### CHARACTERIZATION OF HIV-2 ROD AND HIV-2 EHO ENVELOPE PRECURSORS

There are at least 30 potential N-linked glycosylation sites on the envelope precursor of HIV-2 ROD. In view of this, it remained possible that the 20 kDa difference between the envelope precursor of HIV-2 EHO and ROD might be due to a reduced number of oligosaccharide chains in the envelope precursor of HIV-2 EHO. For this reason, the electrophoretic mobilities of these precursors was analysed after deglycosylation by β-N-acetylglucosaminase H (endo H), which cleaves high mannose type oligosaccharide chains (Tarentino et al., 1974 H.J.Biol. Chem. 249, 818-824). HIV-2 EHO or ROD infected cells were labeled with [³⁵S] methionine and cell extracts were used for the purification of the dimeric forms of envelope precursors by immunoprecipitation and preparative gel electrophoresis. The purified dimer precursors were then dissociated in the presence of SDS and acidic pH (Rey et al., 1989a) before digestion with endo H. the apparent molecular weights of the envelope precursors gp140 (ROD) and gp120 (EHO) were reduced to proteins of 80 and 60 kDa, respectively (Figure 3). These results therefore indicated that the 20 kDa difference between the envelope precursors of the two HIV-2 isolates was not due to a difference in the number of N-linked oligosaccharide chains. Under the experimental conditions of these experiments, digestion with endo-H resulted in a complete cleavage of all oligosaccharide chains.

In order to compare the polypeptide pattern of the envelope precursors of HIV-2 ROD and EHO, we carried out partial proteolysis experiments with Staphyloccocus aureus V8 protease, which manifests specificity for glutamyl bonds. The purified envelope-dimer precursors were first dissociated, before partial proteolysis with different concentrations of the V8 protease. The results obtained with 1µg of the protease are shown in Figure 4. The gp 140 arising from the dissociation of gp300 of HIV-2 ROD was converted to three major polypeptides of 130, 110 and 80 kDa whereas, the gp120 arising from the dissociation of gp270 of HIV-2 EHO was converted to a major 60 kDa polypeptide and two faint 90 and 75 kDa polypeptides. Thus digestion of gp140 and gp120 with the V8 protease resulted in the production of distinct polypeptide patterns which reflected differences in the amino-acid composition between these two envelope precursors.

### HIV-2 EHO ENVELOPE GLYCOPROTEINS ARE NOT RECOGNIZED BY MURINE POLYCLONAL ANTIBODIES AGAINST THE ENVELOPE GLYCOPROTEINS OF HIV-2 ROD.

Polyclonal antibodies were prepared by immunizing mice with a purified preparation of gp300 from cells infected with HIV-2 ROD ("Materials and Methods"). These antibodies were referred to as anti-gp300 polyclonal antibodies. The reactivity of these antibodies was tested in an immunoprecipitation assay using extracts from HIV-1 BRU, HIV-2 ROD, HIV-2 EHO and SIV-mac infected cells labeled with [³H] glucosamine (Figure 5). These assays were carried out in parallel with typical human sera specific for HIV-1 and HIV-2. Anti-gp300 polyclonal antibodies immunoprecipitated gp300, gp140, gp125 and gp80 of HIV-2 ROD with a similar affinity as the HIV-2 positive human serum. In contrast, anti-gp300 antibodies did not recognize any of the envelope glycoproteins of HIV-2 EHO (Figure 5, sections ROD and EHO). However, these latter (gp270, gp120, gp100 and gp80) were immunoprecipitated by the typical HIV-2 positive human serum. These observations indicate the presence of similar and some distinct epitopes on both the extracellular and the transmembrane regions of the envelope glycoproteins of HIV-2 EHO (Figure 5, sections ROD AND EHO).

Anti-gp300 antibodies did not recognize HIV-1 envelope glycoproteins. The precursor gp160 and the extracellular glycoprotein gp120, which as expected, were immunoprecipitated by the HIV-1 specific human serum (Figure 5, section HIV-1). Interestingly, anti-gp300 antibodies immunoprecipitated SIV-mac envelope glycoproteins : the envelope precursor gp140, the precursor dimer gp300 and the extracellular glycoprotein gp130. These SIV-mac glycoproteins were also immunoprecipitated by the HIV-2 specific human serum (Figure 5 section SIV-mac). The reactivity of anti-gp300 antibodies with envelope glycoproteins of both HIV-2 ROD and SIV-mac but not with those of HIV-2 EHO, confirms the important homology between HIV-2 ROD and SIV-mac and also suggests that HIV-2 EHO belongs to a subtype of the HIV-2 that could be differentiated by its lower homology to both HIV-2 ROD and SIV-mac.

Anti-gp300 antibodies along HIV-2 and HIV-1 specific human sera were used in an immunoblot assay with extracts from HIV-1 BRU, HIV-2 ROD and HIV-2 EHO infected cells (Figure 6). The HIV-2 positive serum identified strongly gp300, gp140 and gp80 of HIV-2 ROD, and gp270, gp120 and gp80 of HIV-2 EHO but it showed no reactivity with envelope glycoproteins of HIV-1 (Figure 5, section Serum HIV-2 ; 10% polyacrylamide gel). The poor affinity of this serum with the extracellular glycoprotein of HIV-2 was probably due to low reactivity with the denatured protein since this same serum could identify gp125 (ROD) and gp100 (EHO) under native conditions of the immunoprecipitation assay (Figure 5). Anti-gp300 antibodies identified strongly all the envelope glycoprotein of HIV-2 ROD (gp300, gp140, gp125 and gp80) but it showed no reactivity with the corresponding envelope glycoproteins of HIV-2 EHO nor with proteins of HIV-1 (Figure 6, section anti-gp300 ; 7.5% polyacrylamide gel). The reactivity of anti-gp300 antibodies with the 60kDa protein was found to be not specific because it was observed in cell extracts independent of virus infection. These results indicate that anti-gp300 antibodies do not react with envelope glycoproteins of HIV-2 EHO, either native or denatured form.

Comparison of the results obtained by immunoblot analysis and the immunoprecipitation assays (Figures 5 and 6), show that patient serum and anti-gp300 polyclonal antibodies recognize the denatured form of gp80 better than its native form. The native form of this transmembrane glycoprotein-dimer probably has a conformation which masks the epitopes identified by these different antibodies.

### THE CROSS-REACTIVITY OF AN HIV-1 POSITIVE SERUM WITH DENATURED ENVELOPE GLYCOPROTEINS OF HIV-2 ROD BUT NOT HIV-2 EHO.

In these experiments, an HIV-1 positive human serum was used, which in an immunoprecipitation assay manifested a specificity towards HIV-1 extracellular envelope glycoprotein gp120. In an immunoblot assay, this serum identified gp120 and gag precursors p55 and P40. In addition, this HIV-1 positive serum reacted strongly with gp300 and gp80 of HIV-2 ROD whereas no reactivity was observed with HIV-2 EHO envelope glycoproteins (Figure 6, section Serum HIV-1 ; 10% polyacrylamide gel). This observation indicates that there might be some cross-reactivity between few HIV-1 positive sera and HIV-2 envelope glycoproteins. This cross-reactivity seems to be directed towards the dimeric forms of the envelope, i.e., the dimeric form of the envelope precursor gp300 and the transmembrane glycoprotein dimer gp80. The monomeric form of the precursor gp140 was very weakly recognized by this HIV-1 positive serum, whereas the extracellular glycoprotein gp125 was not at all recognized. In view of these, it is possible to suggest that HIV-1 positive serum recognized an epitope in the transmembrane region of the HIV-2 ROD envelope glycoprotein and for this interaction both denaturation and dimerization were required. These results emphasize antigenic differences between the envelope glycoproteins of HIV-2 ROD and EHO.

The following table 1 gives the results of the comparison between the different retroviruses HIV-1 and HIV-2 and isolate of the latter.

**Table 1**

| Comparative molecular weight (kDa) of viral proteins of HIV-1 BRU, HIV-2 ROD, HIV-2 EHO, SIVmac | | | | | | | | |
|---|---|---|---|---|---|---|---|---|
| VIRUS | ENV | | | | | GAG | | VPX |
| | Pr | | EC gp | TM gp | | Pr | MP | X |
| | Monomer | Dimer | | Monomer | Dimer | | | |
| HIV-1 BRU | 160 | - | 120 | 41 | - | 55 | 25 | - |
| HIV-2 ROD | 140 | 300 | 125 | 36 | 80 | 55 | 26 | 16 |
| HIV-2 EHO | 120 | 270 | 100 | 36 | 80 | 56 | 27 | 14 |
| SIV mac | 140 | 300 | 130 | 32 | 65 | 56 | 28 | 14 |
| Pr : Preursor EC gp : Extracellular glycoprotein TM gp : Transmembrane glycoprotein MP : Major Protein of the core | | | | | | | | |

## Claims

1. Immunogenic composition comprising a purified antigen constituted by the envelope glycoprotein of HIV-2 EHO or a glycoprotein of a variant thereof or part of said antigen, or an antigen having the same immunological properties, which antigen is recognizable by human HIV-2 positive serum which is capable of recognizing the antigens of HIV-2 ROD in an immunoprecipitation assay or in a western blot assay and which antigen is not recognized by antibodies directed against the glycoprotein gp300 of HIV-2 ROD in an immunoprecipitation assay or in a western blot assay under the same conditions.

2. Immunogenic composition according to claim 1 wherein the purified antigen is encoded by the env gene and has a molecular weight of the order of 120 kDa corresponding to the envelope precursor.

3. Immunogenic composition according to claim 1 or 2, wherein the purified antigen is a glycoprotein gp270 corresponding to a dimeric form of the gp120 and in that it has a molecular weight of the order of 270 kDa.

4. Immunogenic composition according to claim 1, wherein the purified antigen corresponds to the mature product of the precursor gp120 and is the extracellular envelope glycoprotein gp100 having a molecular weight of the order of 100 kDa.

5. Immunogenic composition according to claim 1, wherein the purified antigen is a glycoprotein gp36, having a molecular weight of the order of 36 kDa or a glycoprotein gp80 corresponding to the dimeric from of the gp36.

6. Immunogenic composition according to anyone of claims 1 to 5, comprising a non-glycosylated form of the envelope glycoprotein of HIV-2 EHO or a non-glycosylated form of a glycoprotein of a variant thereof, or part of said non-glycosylated antigen or an antigen recognizable by the serum of a patient infected by HIV-2 EHO.

7. Immunogenic composition according to claim 1 or 4, further comprising one or more of the glycoproteins gp270, gp120, gp180 or gp36 of HiV-2 EHO.

8. Immunogenic composition according to anyone of claims 1 to 7, further comprising at least one antigen of HIV-2 ROD, especially antigens chosen among gp300, gp140, gp125, gp80 or gp36.

## Patentansprüche

1. Immunogene Zusammensetzung, enthaltend ein gereinigtes Antigen, das aus dem Hüll-Glycoprotein von HIV-2 EHO oder einem Glycoprotein von einer Variante, oder einem Teil des Antigens gebildet ist, oder ein Antigen mit den gleichen immunologischen Eigenschaften, wobei das Antigen von einem humanen HIV-2-positiven Serum erkannt werden kann, welches zur Erkennung der Antigene von HIV-2 ROD in einem Immunpräzipitationstest oder einem Western-Blot-Test in der Lage ist, und wobei das Antigen von Antikörpern, die gegen das Glycoprotein gp300 von HIV-2 ROD gerichtet sind, in einem Immunpräzipitationstest oder einem Western-Blot-Test unter den gleichen Bedingungen nicht erkannt wird.

2. Immunogene Zusammensetzung nach Anspruch 1, worin das gereinigte Antigen von env-Gen kodiert wird und ein Molekulargewicht in der Größenordnung von 120 kDa, entsprechend dem Hüll-Vorläufer, hat.

3. Immunogene Zusammensetzung nach Anspruch 1 oder 2, worin das gereinigte Antigen ein Glycoprotein gp270 entsprechend einer dimären Form von gp120 ist, und daß es ein Molekulargewicht in der Größenordnung von 270 kDa hat.

4. Immunogene Zusammensetzung nach Anspruch 1, worin das gereinigte Antigen dem reifen Produkt des Vorläufers gp120 entspricht, und das extrazelluläre Hüll-Glycoprotein gp100 mit einem Molekulargewicht in der Größenordnung von 100 kDa ist.

5. Immunogene Zusammensetzung nach Anspruch 1, worin das gereinigte Antigen ein Glycoprotein gp36 mit einem Molekulargewicht in der Größenordnung von 36 kDa, oder ein Glycoprotein gp80, entsprechend der dimeren Form des gp36 ist.

6. Immunogene Zusammensetzung nach einem der Ansprüche 1 bis 5, umfassend eine nicht-glycosylierte Form des Hüll-Glycoproteins von HIV-2 EHO oder eine nicht-glycolysierte Form eines Glycoproteins von einer Variante, oder ein Teil des nicht-glycosylierten Antigens, oder ein Antigen, das durch das Serum eines mit HIV-2 EHO infizierten Patienten erkannt werden kann.

7. Immunogene Zusammensetzung nach Anspruch 1 oder 4, die ferner eines oder mehrere der Glycoproteine gp270, gp120, gp180 oder gp36 von HIV-2 EHO enthält.

8. Immunogene Zusammensetzung nach einem der Ansprüche 1 bis 7, die ferner mindestens ein Antigen von HIV-2 ROD, insbesondere Antigene, ausgewählt aus gp300, gp140, gp125, gp80 oder gp36 enthält.

## Revendications

1. Composition immunogénique comprenant un antigène purifié constitué par la glycoprotéine d'enveloppe de HIV-2 EHO ou une glycoprotéine d'un variant de celui-ci ou une partie dudit antigène, ou un antigène ayant les mêmes propriétés immunologiques, lequel antigène est reconnaissable par le sérum humain positif pour HIV-2, capable de reconnaître les antigènes de HIV-2 ROD dans un test d'immunoprécipitation ou dans un dosage Western-blot et lequel antigène n'est pas reconnu par des anticorps dirigés contre la glycoprotéine gp300 de HIV-2 ROD dans un test d'immunoprécipitation ou dans un dosage Western-blot dans les mêmes conditions.

2. Composition immunogénique selon la revendication 1, caractérisée en ce que l'antigène purifié est codé par le gène env et a un poids moléculaire de l'ordre de 120 kDa correspondant au précurseur d'enveloppe.

3. Composition immunogénique selon la revendication 1 ou 2, caractérisée en ce que l'antigène purifié est une glycoprotéine gp270 correspondant à une forme dimère de gp120 et en ce qu'il a un poids moléculaire de l'ordre de 270 kDa.

4. Composition immunogénique selon la revendication 1, caractérisée en ce que l'antigène purifié correspond au produit mature du précurseur gp120 et il est la glycoprotéine d'enveloppe extracellulaire gp100 ayant un poids moléculaire de l'ordre de 100 kDa.

5. Composition immunogénique selon la revendication 1, caractérisée en ce que l'antigène purifié est une glycoprotéine gp36, ayant un poids moléculaire de l'ordre de 36 kDa ou une glycoprotéine gp80 correspondant à la forme dimère de gp36.

6. Composition immunogénique selon l'une quelconque des revendications 1 à 5, comprenant une forme non glycosylée de la glycoprotéine d'enveloppe de HIV-2 EHO ou une forme non glycosylée d'une glycoprotéine d'un variant de celui-ci, ou une partie dudit antigène non glycosylé ou un antigène reconnaissable par le sérum d'un patient infecté par HIV-2 EHO.

7. Composition immunogénique selon la revendication 1 ou 4, comprenant en outre une ou plusieurs des glycoprotéines gp 270, gp120, gp180 ou gp36 de HIV-2 EHO.

8. Composition immunogénique selon l'une quelconque des revendications 1 à 7, comprenant en outre au moins un antigène de HIV-2 ROD, notamment des antigènes choisis parmi gp300, gp140, gp125, gp80 ou gp36.
